# EUROPÄISCHE PATENTSCHRIFT

(11) **EP 2 141 128 B1**
(45) Veröffentlichungstag und Bekanntmachung des Hinweises auf die Patenterteilung: **27.03.2013**
(21) Anmeldenummer: 09008731.3
(22) Anmeldetag: 03.07.2009
(51) Int. Cl.: B09B 3/00, C02F 11/04

(54) **Verfahren zur Behandlung von Mischsubstanzen in Biogasanlagen**
Method for treating mixed substances in biogas systems
Procédé de traitement de substances mélangées dans des installations de biogaz

(30) Priorität: 04.07.2008 DE 102008032041
(43) Veröffentlichungstag der Anmeldung: 06.01.2010
(73) Patentinhaber: Holm, Niels Christian, 32425 Minden (DE); Rönner-Holm, Sabine, 32425 Minden (DE)
(72) Erfinder: Holm, Niels Christian, 32425 Minden (DE); Rönner-Holm, Sabine, 32425 Minden (DE)
(74) Vertreter: Lobemeier, Martin Landolf

(56) Entgegenhaltungen:
- US-A- 4 328 104

## Beschreibung

Die Erfindung betrifft ein Verfahren zur gleichzeitigen Behandlung unterschiedlicher Ausgangssubstrate in Biogasanlagen zur Biogaserzeugung, wobei durch eine geeignete Prozessführung die hydraulischen Verweildauern der unterschiedlichen Substrate im reaktiven Teil der Biogasanlage auf ihre jeweils erforderlichen unterschiedlichen Gärverweilzeiten ausgerichtet sind.

Anlagen zur Behandlung von Schlämmen (z.B. Primär- oder Überschussschlämmen aus Kläranlagen), Gülle von Rindern, Schweinen oder Hühnern oder auch so genannte Nawaros (nachwachsende Rohstoffe wie Mais, Getreide etc.) werden üblicherweise für Verweildauern ausgelegt, die bei mesophilen Anlagen ca. 20 - 100 Tage und bei thermophilen Anlagen ca. 10 - 50 Tagen betragen.

Die großen Unterschiede in den erforderlichen Verweildauern sind bedingt durch unterschiedlich schnelle Umsatzraten der Ausgangsubstrate. Im Allgemeinen gilt, dass flüssige Ausgangssubstrate (wie z.B. Gülle) in Biogasanlagen unter anaeroben Bedingungen schneller umgesetzt werden als feste Ausgangssubstrate wie z.B. Maissilage, da partikuläre Substanzen erst aufgeschlossen werden müssen.

Um das Ausmaß der Ausgärung zu maximieren, werden Biogasanlagen meistens 2-stufig mit einem vorgeschalteten Gärbehälter (in den alle Ausgangsubstrate hineingefördert werden) und einem nachgeschalteten Nachgärbehälter, der aus dem Gärbehälter beschickt wird, gebildet. Diese 2-stufige Prozessführung ist auf Grund des höheren Raumbedarfs im Gärbehälter und entsprechend geringeren Raumbedarfs im Nachgärbehälter hinsichtlich einer Maximierung der Ausgärung vorteilhaft. Die gesamte mittlere Verweildauer ergibt sich dabei aus dem Verhältnis der Menge an Ausgangssubstraten zum Gesamtvolumen Gärbehälter und Nachgärbehälter.

Die Größenauslegung von Biogasanlagen erfolgt bei nur einem Ausgangssubstrat auf die erforderliche Verweilzeit eben dieses Substrates. Bei der Behandlung unterschiedlicher Substrate mit unterschiedlich erforderlichen Verweilzeiten erfolgt die Größenauslegung entweder:
1. auf Basis des Substrates mit der höchsten erforderlichen Verweilzeit oder
2. auf Basis einer Mischkalkulation

Strategie 1. hat den Nachteil, dass die Anlage größer wird als für alle Ausgangsubstrate erforderlich und Strategie 2 hat den Nachteil, dass das Substrat, das die höchste Verweilzeit erfordert, nicht gänzlich ausgegoren wird.

In der WO 2005/000 748 A1 wird im Stand der Technik eine Biogasanlage offenbart, bei der Tierdung nach Anmischen und Erwärmen direkt in einen Reaktor eingeleitet wird. Zudem kann ein weiteres Substrat direkt in den Reaktor eingeleitet werden. Dagegen wird zerkleinertes Strohmaterial in einem anaeroben Hydrolyse-Tank unter Druck behandelt, bevor es in den Reaktor eintritt. Ein Hydrolyse-Tank ist jedoch wegen der dort herrschenden hohen Temperaturen von bis zu 200 °C nur als Vorstufe nicht selbst für die Ausgärung der Substrate geeignet, die länger gären sollen.

Weiter wurde bereits über eine Entkopplung der Verweilzeit der organischen Feststoffpartikel aus organischen Reststoffen aus häuslicher Herkunft von den der gelösten Stoffe in Abwässern mittels einer Trennvorrichtung in der DE 20 2004 010 707 Ul ein Vorschlag gemacht. Das dort verwendete Siebblech ist jedoch nicht in der Lage im großen Maßstab die erforderliche Trennung zu bewirken. Es hält entweder zu viel zurück (zu kleiner Maschen, die sich dicht setzen) oder es gelangen zuviel des eigentlich zurückzuhaltenden Substrats mit in eine innere Methanisierungszone.

Dieser zweistufigen Reaktor besteht zudem aus einer Hydrolyse- sowie einer Versäuerungszone und Methanisierungszone wobei die beiden koaxial angeordneten Kammern nur durch ein Sieb getrennt. Abwässer und Reststoffe werden daher auch zurücklaufen, nachdem eine Lösung in die innere Methanisierungszone eingetreten ist.

Gemäß der schließlich noch zu nennenden DE 199 46 299 C2 werden von schwer und leicht abbaubaren Bioabfällen getrennt Suspensionen bereitet, die in getrennten Behältern behandelt werden. In einem Enzymbehälter werden z.B. Papier und Molke zusammen mit nicht abgebautem Sediment aus einem (zweiten) Hydrolysebehälter gemischt und vorbehandelt, in einem (ersten) Hydrolysebehälter mikrobiell abgebaut und anschließend dem zweiten Hydrolysebehälter zugeführt. Erst dort erfolgt eine Vermischung mit dem Massenstrom der leicht abbaubaren Stoffe. Von dem zweiten Hydrolysebehälter gelangen die Stoffe in den Methan- und Gärbehälter.

Somit ist aus dem Stand der Technik lediglich bekannt, leicht und schwer abbaubare Substrate einer unterschiedlichen Vorbehandlung zuzuführen, bevor sie dem Biogasreaktor zugeführt werden.

Ein Hydrolysereaktor kann dabei nicht als Gärbehälter dienen, weil bei den Teilschritten der anaeroben Hydrolyse und Acidogenese noch keine Enzyme zugegeben wurde. Der pH-Wert liegt um 4,5 bis 5.

Die Gärung erfolgt erst nach Abkühlung auf um die 30°C und erst der dem zweiten Hydrolysebehälter entnommene Massestrom enthält die Substrate für eine Methanogenese und man arbeitet wieder bei einem pH-Wert um 7.

Der Erfindung liegt damit die Aufgabe zugrunde, Die Biogaserzeugung aus Maissilage und Gülle durch Volumeneinsparung zu optimieren. Diese Aufgabe wird durch die im Anspruch 1 genannten Merkmale gelöst.

Es wird also vorgeschlagen, dass die Gülle nicht zuerst in den Gärbehälter sondern direkt in den Nachgärbehälter gefördert wird. Für dieses Substrat ergibt sich dadurch eine geringere Verweildauer als die mittlere gesamte Verweildauer, für die Maissilage ergibt sich dagegen eine höhere Verweildauer als die mittlere Verweildauer.

Aus der WO 2008116842 ist gemäß are 54(3) EPÜ ein Verfahren bekannt, bei dem ein erstes Substrat in einem ersten Gärbehälter und dann in einen Nachgärbehälter geführt wird. Ein zweites Substrat (dort Waschwasser) wird direkt in den Nachgärbehälter geführt.

### Beispiel:

### Bisheriges Verfahren:

Eine Biogasanlage zur Behandlung von 50 m³/d Schweinegülle mit 6% TS (erforderliche Verweilzeit 40 Tage) und 20 m³ Maissilage mit 30% TS (erforderliche Verweilzeit 80 Tage) wird auf eine erforderliche Mais-Verweildauer von 80 Tagen ausgelegt, weil das Biogaspotential des Maisanteils viel höher ist als in der Gülle. Es ergibt sich damit ein erforderliches Volumen von 70 x 80 = 5.600 m³ aufgeteilt auf z.B. einen Gärbehälter und einen Nachgärbehälter mit jeweils 2.800 m³ Nutzvolumen. Mit einer mittleren Abbaurate von 75% bezogen auf den TS ergibt sich bei der Entnahme aus dem Nachgärbehälter ein TS im Gärrest von: ((50 x 6) + (20 x 30))/70 x 0,25 = 3,2% TS. Daher kann im Gärbehälter von einem TS - Gehalt von ca. 4% ausgegangen werden.

### Vorgeschlagenes Verfahren:

Die Maissilage wird in den Gärbehälter gefördert, die Gülle dagegen in den Nachgärbehälter. Die Entnahme aus dem Gärbehälter erfolgt in den Nachgärbehälter, so dass sich für den Mais die volle Verweildauer Gär- + Nachgärbehälter ergibt und für die Gülle "nur" die Verweildauer auf Basis des Nachgärbehälters. Mit einer mittleren Abbaurate von 75% bezogen auf den Mais TS ergibt sich bei der Entnahme aus dem Gärbehälter ein TS im Gärrest von: 30 x 0,25 = 7,5% TS. Die Verweildauer der Maissilage in einem 2.800 m³ Gärbehälter beträgt somit 2.800/20 = 140 Tage. Die Verweildauer von Mais + Gülle in einem nachfolgenden 2.800 m³ Nachgärbehälter beträgt dann 2.88/(20 + 50) = 40 Tage. Mit diesem Verfahren beträgt die Verweildauer von Mais somit insgesamt 180 Tage und von Gülle insgesamt 40 Tage.

Auf 80 Tage Maisverweildauer ausgelegt müsste die Aufenthaltszeit im Gärbehälter somit nur 40 Tage betragen: 40 x 20 = 800 m³ Nutzvolumen Gärbehälter. Mit diesem Verfahren kann somit bezogen auf dieses konkrete Beispiel 2.000 m³ Gärbehälter Nutzvolumen eingespart werden. Das hinsichtlich Gülle somit nicht gänzlich genutzte Aufenthaltsvolumen wird um ein Vielfaches ausgeglichen durch die verlängerte Aufenthaltszeit der Maissilage, die sich letztendlich überwiegend über den erhöhten TS - Gehalt im Gärbehälter ergibt.

Bei diesem Beispiel ist eine exakte Einstellung der Verweildauern möglich über eine entsprechende Volumenaufteilung des Gär- und Nachgärbehälters bei ausschließlicher Zufuhr von Substrat 1 (hier Maissilage) in den Gärbehälter und Substrat 2 (Schweinegülle) in den Nachgärbehälter.

Eine solche exakte Einstellung ist insbesondere bei mehr als zwei Substraten nicht immer möglich bzw. wirtschaftlich/technisch sinnvoll. Entscheidend ist, dass gewährleistet wird, dass das Substrat mit dem höchsten Biogaspotential und der höchsten erforderlichen Verweilzeit auf diese hohe Verweilzeit ausgelegt werden kann, ohne dass das erforderliche Gesamtvolumen auf Grund anderer Substrate unwirtschaftlich groß wird.

Zur Maximierung der Volumeneinsparung bei drei (oder mehr) unterschiedlichen Ausgangssubstraten kann eine dritte Gärstufe (nach dem 2. Gärbehälter) für ein drittes Substrat (oder Substrate) mit einer kürzeren Verweildauer als Gülle das direkt in den 2. Gärbehälter geführt wird vorgesehen werden.

Andererseits wird vorgeschlagen zur Maximierung der Verweilzeit bei drei (oder mehr) unterschiedlichen Ausgangssubstraten dann eine Gärstufe (nach einem 1. oder 2. Gärbehälter) für ein Substrat vorzusehen, das direkt in diesen Nachgärbehälter geführt wird, wenn ein wesentlicher Unterschied der Verweilzeiten zwischen Maissilage und drittem Substrat vorhanden ist.

Dabei wird zur Maximierung der Verweilzeit des Substrats mit dem höchsten Biogaspotential und der höchsten erforderlichen Verweilzeit eine hohe Verweilzeit bei der Auslegung der Parameter bestimmend sein, während die weiteren Verweilzeiten der anderen Substrate zur Bestimmung des erforderlichen Gesamtvolumen dienen.

## Patentansprüche

1. Verfahren zur Biogaserzeugung aus Maissilage und Gülle, wobei die Gülle eine höhere Umsatzrate als die Maissilage aufweist, durch Betreiben einer mit einem Gärbehälter und einem diesem nachgeschalteten Nachgärbehälter versehenen Biogasanlage,
mit den Schritten:
Fördern der Maissilage in den Gärbehälter für eine erste Verweildauer,
Entnehmen der Maissilage aus dem Gärbehälter nach der ersten Verweildauer und Fördern der Maissilage in den Nachgärbehälter,
Fördern der Gülle in den Nachgärbehälter und
Verweilen lassen von Maissilage und Gülle im Nachgärbehälter für eine zweite Verweildauer.

## Claims

1. A method for biogas production from maize silage and liquid manure, the liquid manure having a greater conversion rate than maize silage, by operating a biogas plant equipped with a digestion tank and a post-digestion tank downstream therefrom,
having the steps:
conveying the maize silage into the digestion tank for a first dwell period,
removing the maize silage from the digestion tank after the first dwell period and conveying the maize silage into the post-digestion tank,
conveying the liquid manure into the post-digestion tank and
leaving the maize silage and the liquid manure in the post-digestion tank for a second dwell period.

## Revendications

1. Procédé pour la production de biogaz à partir de sillage de maïs et lisier, étant entendu que le lisier présente un taux de rendement plus élevé que le sillage de maïs, par l'exploitation d'une installation de biogaz, pourvu d'un digesteur et d'un post-digesteur placé en aval,
avec les étapes :
transport du sillage de maïs dans le digesteur pour un premier temps de résidence,
enlèvement du sillage de maïs du digesteur après la première durée de séjour et transport du sillage de maïs dans le post-digesteur,
transport du lisier dans le post-digesteur et
séjour du sillage du maïs et lisier dans le post-digesteur, pour une deuxième durée de séjour.
